# EUROPEAN PATENT APPLICATION

(11) **EP 4 570 816 A1**
(43) Date of publication of application: **18.06.2025**
(21) Application number: 23383298.9
(22) Date of filing: 14.12.2023
(51) Int. Cl.: C07K 14/435, A61K 38/17, A61P 31/14

(54) **SOLUBLE GP130FC (SGP130FC) FOR USE IN THE TREATMENT OF SARS-COV-2**

(71) Applicant: Consejo Superior De Investigaciones Científicas - CSIC, 28006 Madrid (ES)
(72) Inventor: BUSTOS DE ABAJO, MATILDE, Sevilla (ES); RODRIGUEZ HERNANDEZ, MARIA ANGELES, Sevilla (ES); ROSE-JOHN, STEFAN, Kiel (DE)
(74) Representative: Pons

(57) **Abstract**

The present invention relates to a specific inhibitor of IL-6 trans-signaling, the protein sgp130Fc, for use in the treatment or prevention of a disease caused by coronavirus infection in a subject, preferably caused by SARS-CoV-2.

## Description

The present invention belongs to the technical field of medicine, particularly for the treatment of diseases caused by virus. The present invention relates to a specific inhibitor of IL-6 trans-signaling, the protein sgp130Fc, for use in the treatment of SARS-CoV-2 disease.

### BACKGROUND ART

Coronaviruses are a large family of RNA viruses that generally cause mild to moderate upper respiratory tract disease in humans and a wide variety of animals, including mammals and birds. However, three coronaviruses have caused more severe and fatal disease in humans: the SARS coronavirus (SARS-CoV), which emerged in November 2002 and causes severe acute respiratory syndrome (SARS); the MERS coronavirus (MERS-CoV), which emerged in 2012 and causes Middle East respiratory syndrome (MERS); and the SARS-CoV-2 severe respiratory syndrome coronavirus type 2 SARS-CoV-2, which emerged in 2019 and causes coronavirus disease 2019 (COVID-19).

Severe stage COVID19 causes a hyperinflammatory syndrome culminating in respiratory dysfunction and multi-organ damage. That is, SARS-CoV-2 infection induces activation of proinflammatory signaling in lung epithelial cells expressing high levels of ACE2 and TMPRSS2, leading to a dysregulated pulmonary immune response and systemic inflammatory response syndrome (SIRS) or also known as "cytokine storm", i.e., massive production of proinflammatory cytokines, altering the interaction of epithelial cells and immune cells (Jackson et al., Nature reviews Molecular cell biology, 23(1), 3-20. January 2022). The proinflammatory cytokines involved in this phenomenon are tumor necrosis factor alpha (TNF-α), interleukin 1 beta (IL-1β) and interleukin 6 (IL-6). The latter has received much attention since the beginning of the pandemic because of its therapeutic potential. Interleukin (IL)-6 and soluble IL-6 receptor (slL-6Rα) were identified among the key players in the COVID-19-induced cytokine release syndrome (Felsenstein, et al., Clin Immunol, 215:108448. June 2020).

IL-6 is a cytokine that is involved in a number of processes including inflammation, acute phase response, infection, neurodegenerative processes, autoimmunity and trauma. IL-6 is involved in both anti-inflammatory and proinflammatory responses. The cytokine exerts its effects through different mechanisms depending on the target cells. On the one hand, classical IL-6 signaling, which mainly controls acute inflammatory responses (anti-inflammatory effects), such as the acute phase reaction, is induced by IL-6 binding to the membrane-bound IL-6Rα on target cells. This event is followed by recruitment of the ubiquitously expressed signal transducer glycoprotein 130 (gp130) co-receptor and the initiation of downstream signaling cascades leading to activation of the signal transducer/Janus kinase and activator of transcription (JAK/STAT), protein kinase B (PKB or AKT) and mitogen-activated protein kinase (MAPK) pathway. On the other hand, IL-6 trans-signaling allows cells lacking the membrane-bound IL-6Rα to be stimulated by IL-6. This mode of signaling is considered causative for the proinflammatory properties, with a key role in many chronic inflammatory diseases, including rheumatoid arthritis, multiple sclerosis, inflammatory bowel disease and cancer. IL-6 trans-signaling occurs through the formation of IL-6 complexes with soluble forms of IL-6Rα (sIL-6Rα). These complexes can activate downstream signaling cascades in all cells expressing gp130. In the third way of IL-6 signaling, cluster signaling (also termed trans-presentation), IL-6 is presented bound to the IL-6Rα on the surface of dendritic cells to cognate T cells, which are activated via gp130 homodimerization. This mechanism is crucial for the priming of pathogenic Th17 cells but might also have additional roles yet to be discovered (Giraldez, et al., Nat Rev Gastroenterol Hepatol, 18(11): p. 787-803. November 2021).

Targeting IL-6 signaling is of interest for many diseases. However, global blockade of IL-6, e.g., through antibodies against IL-6, is associated with many side effects (including an increased risk of bacterial and viral infections) due to the multitude of functions of this cytokine. Therefore, to efficiently target different disease-specific IL-6 functions, it is of utmost importance to understand the signaling mode of IL-6 in the disease process and the ability to target only this specific signaling mode. The selective inhibitor of IL-6 trans-signaling sgp130Fc, which bound IL-6 only in the presence of slL-6Rα and consequently without affecting classic signaling, has shown therapeutic potential in various preclinical models of disease (Rose-John, et al., Nat Rev Immunol, 2023: p. 1-16. April 2023). New sgp130Fc variants have emerged as a great promise for therapy such as Olamkicept for inflammatory bowel disease, in clinical trials (Schreiber et al., Gastroenterology; 160(7):2354-2366 e2311. June 2021; Zhang et al., JAMA, 329(9):725-734. March 2023).

IL-6 trans-signaling has emerged as the pathological mode of signaling underlying various inflammatory conditions such as autoimmune diseases, sepsis, cytokine release syndrome and other chronic conditions (Anaka et al., Immunotherapy, 8(8): p. 959-70. July 2016*;* Cronstein, B.N., Bull NYU Hosp Jt Dis, 65 Suppl 1: p. S11-5. 2007*;* Jevnikar et al., J Allergy Clin Immunol, 143(2): p. 577-590. June 2018*;* Raita et al., Front Med (Lausanne), 8: p. 665057. April 2021). Emerging evidence implies that IL-6 trans-signaling plays a pivotal role in the pathophysiology of inflammation, coagulopathy, endothelial damage and thrombosis that characterize severe cases of COVID-19. While the focus to date has been on global blockade of classical IL-6 and trans-signaling with tocilizumab and sarilumab in COVID-19 with beneficial effects on survival rates of severe COVID-19 in preclinical and clinical studies, selective blockade of trans-signaling is an area of investigation. In COVID-19, IL-6 receptor antagonists, such as tocilizumab, sarilumab reduced inflammatory responses and improve clinical outcomes in COVID-19 patients (Ghosn et al., Cochrane Database Syst Rev, 3(3): p. CD013881. March 2021*;* Chen et al., Exp Ther Med, 21(1): p. 24. January 2021*;* Abidi et al., Front Pharmacol, 13: p. 825749. February 2022*;* Albuquerque et al., Clin Microbiol Infect, 29(1): p. 13-21. January 2023).

These antibodies prevent IL-6 binding to IL-6Rα and inhibit classical and trans signaling equally well (Kang, S., et al., Immunity, 50(4): p. 1007-1023. April 2019). Since classical IL-6 signaling is considered beneficial and IL-6 is required to control viral infection (Kopfetal., Nature, 368(6469): p. 339-42. March 1994), global blockade of IL-6 signaling is associated with an increased risk of respiratory tract infections and may have the potential to increase viral transmission (Pawar et al., Ann Rheum Dis, 78(4): p. 456-464. April 2019). JAK inhibitors, such as baricitinib, have shown good responses in severe cases of COVID-19 (Kalil and Stebbing, Lancet Respir Med, 9(12): p. 1349-1351 December 2021*;* Marconi et al., Lancet Respir Med, 9(12): p. 1407-1418. December 2021). However, a potential limitation of the use of these anti-inflammatory therapies is the common problem of increased frequency of severe infections. In addition, JAKs are not exclusively activated by IL-6, so targeting these molecules does not allow selective inhibition of IL-6 signaling (Rose-John, et al., Nat Rev Immunol, 2023: p. 1-16. April 2023*;* Jostock et al., Eur J Biochem, 268(1): p. 160-7. January 2001). This could be detrimental to treatment of COVID-19 with IL-6 inhibitors because of a possible increase in viral replication due to IL-6 blockade (WO23012173).

Whereas the antibodies did not differentiate between classical and trans-signaling, the soluble forms of gp130 are selective binders of IL-6:sIL-6Rα complexes, thus only interfering with IL-6 trans-signaling. Indeed, there are preclinical trials blocking IL-6 trans-signaling in animal models of human autoimmune and neoplastic diseases, with clear advantages over global blockade of IL-6 activity. sgp130Fc is the first selective inhibitor of IL-6 trans-signaling, binding IL-6 only in the presence of sIL-6Rα and consequently without affecting classical signaling.

In mice, sgp130Fc prevents death caused by cecal ligation puncture-induced septic shock syndrome and at least bacterial infections are better controllable after selective inhibition of IL-6 trans-signaling compared to classical and trans-signaling inhibition by monoclonal IL-6 antibodies. Therefore, unlike neutralizing antibodies, sgp130Fc, which is supported by mouse experiments, is not expected to affect the control of bacterial and viral infections. A recent Phase II study with sgp130Fc (Olamkicept) (EudraCT, number 2016-000205-36) demonstrated the safety and efficacy of sgp130Fc in the treatment of Crohn's disease and ulcerative colitis (WO23012173).

However, it is necessary to provide an alternative to mitigate both, short- and long-term consequences of COVID-19 and prognosis of lung injury in COVID-19.

### DESCRIPTION OF THE INVENTION

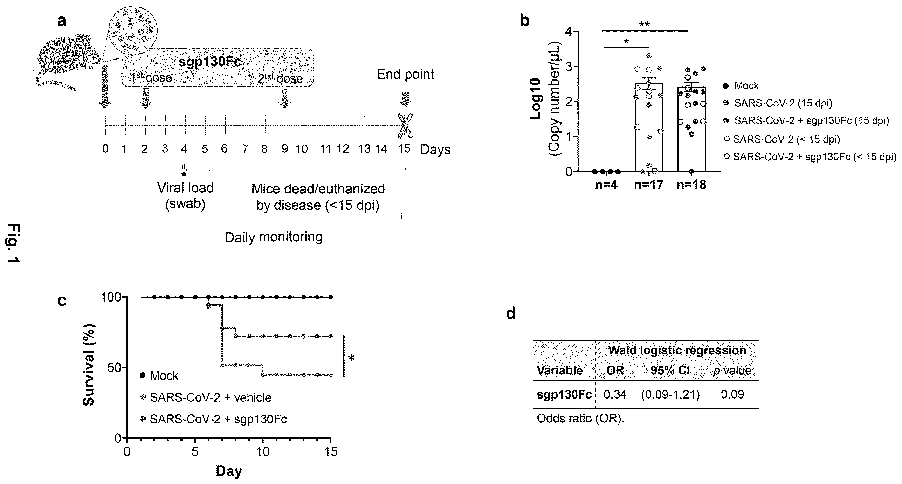

The inventors of the present invention have found evidence of a causal effect on the inhibition of IL-6 trans-signaling on the short- and long-term consequences of SARS-CoV-2 infection *in vivo.* The inventors tested a specific inhibitor of IL-6 trans-signaling, sgp130Fc, in SARS-CoV-2-infected mice and found that sgp130Fc caused a significant decrease in lung injury in surviving mice, supported by analysis of histological and proinflammatory markers (Examples 1 and 2 and Fig. 1 to 3).

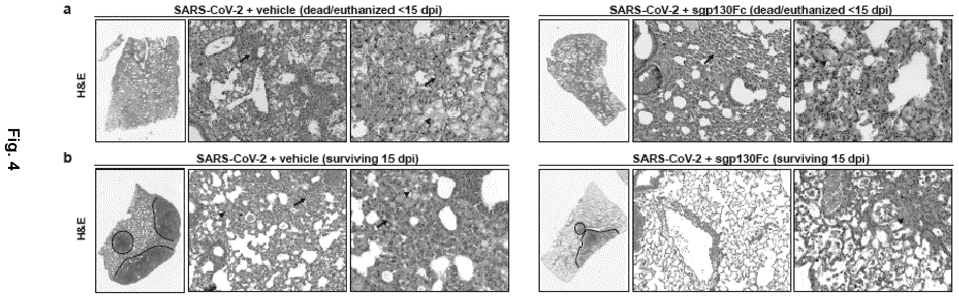

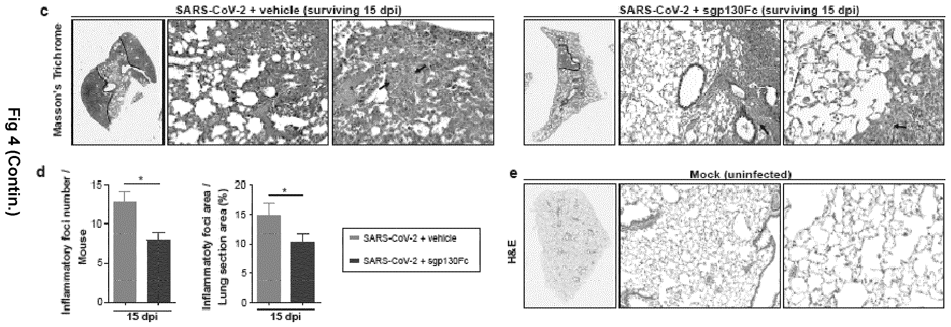

In addition, the inventors also tested the *in vitro* use of interleukin-6 trans-signaling as a molecular target for screening compounds useful in the treatment of a coronavirus infection in a subject, and found that sgp130Fc inhibits IL-6 trans-signaling effects in primary human lung microvascular endothelial cells and lung fibroblasts, showing that IL-6 trans-signaling induces strong and persistent activation of Janus kinase1/signal transducer and activator of transcriptions (JAK1/STAT3) in endothelial cells and lung fibroblasts with proinflammatory effects, partially attenuated by sgp130Fc (Example 3 and 4 and Fig. 4 to 7).

Therefore, these results place IL-6 trans-signaling in the pathogenesis and progression of COVID-19 and suggest that specific blockade of this signaling may offer a promising alternative to mitigate the short- and long-term consequences of COVID-19. These findings have important implications for the treatment and prognosis of lung injury in COVID-19.

In view of the above, a first aspect of the present invention relates to an interleukin-6 trans-signaling inhibitor, hereinafter "inhibitor of the invention", for use in the treatment or prevention of a disease caused by coronavirus in a subject, wherein the Interleukin-6 trans-signaling inhibitor is a fusion protein comprising, in the sense N'terminal to C'terminal, the extracellular region of the glycoprotein 130 and a Fc constant portion of an IgG antibody, hereinafter the "use of the invention".

In the present invention, the inhibitor of the invention is also referred to as sgp130Fc or interleukin-6 trans-signaling inhibitor of the invention.

The terms "interleukin-6 trans-signaling inhibitor" or "the specific inhibitor of IL-6 trans-signaling" as used herein refer to any molecule capable of totally or partially inhibiting the trans-signaling of the cytokine IL-6 by blocking the IL-6 receptor (IL-6Rα). IL-6 is a cytokine that is involved in a number of processes including inflammation, acute phase response, infection, neurodegenerative processes, autoimmunity and trauma. The cytokine exerts its effects through different mechanisms depending on the target cells, for example, in the case of IL-6 trans-signaling, it is observed upregulated or downregulated (pro-inflammatory effects) in many chronic inflammatory diseases, including rheumatoid arthritis, multiple sclerosis, inflammatory bowel disease and cancer. IL-6 trans-signaling occurs through the formation of IL-6 complexes with soluble forms of IL-6Rα (sIL-6Rα). These complexes can activate downstream signaling cascades in all cells expressing glycoprotein 130 (gp130). IL-6 has three binding sites for its receptors. Site I recognizes IL-6Rα, while sites II and III establish contact with gp130. Antibodies against IL-6 or IL-6Rα generally target the site I interface formed between IL-6 and IL-6Rα and affect classical and trans signaling. The fusion protein sgp130Fc targets sites II and III and, in principle, does not affect classical IL-6 signaling, unless extremely high concentrations are used that allow quantitative complexation of IL-6 with sIL-6R and thus may reduce free IL-6 levels. Particularly, IL-6 trans-signaling plays a pivotal role in the pathophysiology of inflammation, coagulopathy, endothelial damage and thrombosis that characterize severe cases of COVID-19.

The protein gp130 (PDB: 8D6A_A) is a transmembrane protein that belongs to the cytokine receptor class, and that comprises an intracellular region, a transmembrane region and an extracellular region. Thus, the extracellular region of the glycoprotein 130, preferably the human glycoprotein gp130, refers to a portion that comprises amino acids of the protein gp130 that are placed on the outside part of the cell.

In a preferred embodiment, the inhibitor of the invention comprises the extracellular region of the glycoprotein 130 that comprises an amino acid sequence having an identity of at least 80 % with the sequence SEQ ID NO: 46.

SEQ ID NO: 46, a fragment of the extracellular region of gp130.

LLDPCGYISPESPVVQLHSNFTAVCVLKEKCMDYFHVNANYIVWKTNHFTIP
KEQYTIINRTASSVTFTDIASLNIQLTCNILTFGQLEQNVYG

In a preferred embodiment, the inhibitor of the invention comprises the extracellular region of gp130 comprises an amino acid sequence having an identity of at least 85%, 90%, 91%, 92%, 93%, 94%, 95%, 96%, 97%, 98%, or 99% with the sequence SEQ ID NO: 46; more preferably, the extracellular region of gp130 comprises the amino acid sequence SEQ ID NO: 46.

In another preferred embodiment, the inhibitor of the invention comprises the extracellular region of the glycoprotein 130 that comprises an amino acid sequence having an identity of at least 80 % with the sequence SEQ ID NO: 47.

SEQ ID NO: 47, a fragment of the extracellular region of gp130:

ELLDPCGYISPESPVVQLHSNFTAVCVLKEKCMDYFHVNANYIVWKTNHFTIPKEQ
YTIINRTASSVTFTDIASLNIQLTCNILTFGQLEQNVYGITIISGLPPEKPKNLSCIVNEG
KKMRCEWDGGRETHLETNFTLKSEWATHKFADCKAKRDTPTSCTVDYSTVYFVNI
EVWVEAENALGKVTSDHINFDPVYKVKPNPPHNLSVINSEELSSILKLTWTNPSIKS
VIILKYNIQYRTKDASTWSQIPPEDTASTRSSFTVQDLKPFTEYVFRIRCMKEDGKG
YWSDWSEEASGITYEDRPSKAPSFWYKIDPSHTQGYRTVQLVWKTLPPFEANGKI
LDYEVTLTRWKSHLQNYTVNATKLTVNLTNDRYLATLTVRNLVGKSDAAVLTIPAC
DFQATHPVMDLKAFPKDNMLWVEWTTPRESVKKYILEWCVLSDKAPCITDWQQE
DGTVHRTYLRGNLAESKCYLITVTPVYADGPGSPESIKAYLKQAPPSKGPTVRTKK
VGKNEAVLEWDQLPVDVQNGFIRNYTIFYRTIIGNETAVNVDSSHTEYTLSSLTSDT
LYMVRMAAYTDEGGKDGPEFTFTTPKFAQGE

The amino acid sequence SEQ ID NO: 47 comprises the amino acid sequence SEQ ID NO: 46.

In another preferred embodiment, the inhibitor of the invention comprises the extracellular region of gp130 that comprises an amino acid sequence having an identity of at least 85%, 90%, 91%, 92%, 93%, 94%, 95%, 96%, 97%, 98%, or 99% with the sequence SEQ ID NO: 47; more preferably, the extracellular region of gp130 comprises the amino acid sequence SEQ ID NO: 47.

As describe above the interleukin-6 trans-signaling inhibitor is sgp130Fc, which is a fusion protein comprising, in the N'-terminal to C'-terminal sense, the extracellular region of human glycoprotein 130 and a constant Fc portion of an Immunoglobulin G (IgG), preferably the Fc of a IgG1 antibody, and more preferably the Fc of human IgG1.

In a preferred embodiment, the inhibitor of the invention comprises a constant Fc portion of an IgG that comprises and amino acid sequence having an identity of at least 80 % with the sequence SEQ ID NO: 48.

SEQ ID NO: 48, constant Fc portion of IgG1:

DKTHTCPPCPAPEAEGAPSVFLFPPKPKDTLMISRTPEVTCVVVDVSHEDP
EVKFNWYVDGVEVHNAKTKPREEQYNSTYRVVSVLTVLHQDWLNGKEYK
CKVSNKALPAPIEKTISKAKGQPREPQVYTLPPSREEMTKNQVSLTCLVKGF
YPSDIAVEWESNGQPENNYKTTPPVLDSDGSFFLYSKLTVDKSRWQQGNV
FSCSVMHEALHNHYTQKSLSLSPGK

The amino acids ranging from residues 1 to 13 in the SEQ ID NO: 48 conform the hinge region of the Fc portion of IgG1 that provides flexibility to the fusion protein comprising it, allowing to correctly perform its inhibition activity. The hinge region included in the constant Fc portion may comprise different amino acid mutations as described in WO2008000516A2, wherein said mutations maintain the flexibility, and do not affect the solubility or inhibition capacity of the fusion protein.

In another preferred embodiment, the inhibitor of the invention comprises a constant Fc portion of an IgG that comprises the amino acid sequence having an identity of at least 85%, 90%, 91%, 92%, 93%, 94%, 95%, 96%, 97%, 98%, or 99% with the sequence SEQ ID NO: 48; more preferably comprises the amino acid sequence SEQ ID NO: 48.

Therefore, the interleukin-6 trans-signaling inhibitor of the invention can be a functionally equivalent variant or fragment of the fusion protein sgp130Fc that comprises the amino acid sequence SEQ ID NO: 1. The amino acid sequence SEQ ID NO: 1 comprises, in the sense N'terminal to C'terminal, the amino acid sequence SEQ ID NO: 46 and SEQ ID NO: 48; more preferably, the amino acid sequence SEQ ID NO: 1 comprises, in the sense N'terminal to C'terminal, the amino acid sequence SEQ ID NO: 47 and SEQ ID NO: 48.

The term "functionally equivalent variant" refers to a protein (or polypeptide) comprising one or more alterations, such as substitutions, insertions, deletions and/or truncations of one or more specific amino acid residues at one or more specific positions in the protein and having the same function that the original protein. The term "functionally equivalent fragment" means a protein or a domain thereof having one or more (e.g., several) amino acids absent from the amino and/or carboxyl terminus of the amino acid sequence, wherein the fragment has the same activity than the whole protein.

Thus, in another preferred embodiment, the Interleukin-6 trans-signaling inhibitor of the invention is a fusion protein comprising an amino acid sequence having a sequence identity of at least 80% with the sequence SEQ ID NO: 1.

SEQ ID NO: 1, amino acid sequence of the Interleukin-6 trans-signaling inhibitor fusion protein sgp130Fc:

ELLDPCGYISPESPVVQLHSNFTAVCVLKEKCMDYFHVNANYIVWKTNHFTIPKEQ
YTIINRTASSVTFTDIASLNIQLTCNILTFGQLEQNVYGITIISGLPPEKPKNLSCIVNEG
KKMRCEWDGGRETHLETNFTLKSEWATHKFADCKAKRDTPTSCTVDYSTVYFVNI
EVWVEAENALGKVTSDHINFDPVYKVKPNPPHNLSVINSEELSSILKLTWTNPSIKS
VIILKYNIQYRTKDASTWSQIPPEDTASTRSSFTVQDLKPFTEYVFRIRCMKEDGKG
YWSDWSEEASGITYEDRPSKAPSFWYKIDPSHTQGYRTVQLVWKTLPPFEANGKI
LDYEVTLTRWKSHLQNYTVNATKLTVNLTNDRYLATLTVRNLVGKSDAAVLTIPAC
DFQATHPVMDLKAFPKDNMLWVEWTTPRESVKKYILEWCVLSDKAPCITDWQQE
DGTVHRTYLRGNLAESKCYLITVTPVYADGPGSPESIKAYLKQAPPSKGPTVRTKK
VGKNEAVLEWDQLPVDVQNGFIRNYTIFYRTIIGNETAVNVDSSHTEYTLSSLTSDT
LYMVRMAAYTDEGGKDGPEFTFTTPKFAQGEDKTHTCPPCPAPEAEGAPSVFLFP
PKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSR
EEMTKNQVSLTCLVKGFYPSDIAVEWESNGQPENNYKTTPPVLDSDGSFFLYSKL
TVDKSRWQQGNVFSCSVMHEALHNHYTQKSLSLSPGK

In the context of the present invention, it is considered that peptides or polypeptides or proteins with an identity of 80% with SEQ ID NO: 1 are functionally equivalent variants of the SEQ ID NO: 1 and maintain the same properties as the sequence to which they refer, i.e., they show interleukin-6 trans-signaling inhibitory activity.

In another preferred embodiment of the invention, the interleukin-6 trans-signalling inhibitor is a fusion protein comprises an amino acid sequence having an identity of at least 85%, 90%, 91%, 92%, 93%, 94%, 95%, 96%, 97%, 98%, or 99% with the sequence SEQ ID NO: 1.

The term "identity" or "sequence identity" as used herein refers to the degree of similarity between two nucleotide or amino acid sequences obtained by aligning the two sequences, i.e. the proportion of identical nucleotide or amino acids between two compared nucleotide sequences or polypeptides/proteins along their full-length sequence. Depending on the number of common residues between the aligned sequences, a different degree of identity, expressed as a percentage, will be obtained. The degree of identity between two amino acids or nucleotides sequences may be determined by conventional methods, for example, by standard sequence alignment algorithms known in the state of the art, such as BLAST [Altschul S.F. et al. Basic local alignment search tool. J Mol Biol. 1990 Oct 5; 215(3): 403-10]. The BLAST programs, for example, BLASTN, BLASTX, and T BLASTX, BLASTP and TBLASTN, are in the public domain at *The National Center for Biotechonology Information (NCBI)* website. The skilled person in the art understands that mutations in the nucleotide sequence of genes that lead to conservative amino acid substitutions at non-critical positions for the functionality of the protein are evolutionarily neutral mutations which do not affect its global structure or its functionality.

In other preferred embodiment of the invention, the interleukin-6 trans-signaling inhibitor is a fusion protein that comprises the amino acid sequence SEQ ID NO: 1.

In other more preferred embodiment of the invention, the interleukin-6 trans-signaling inhibitor is a fusion protein consisting of the amino acid sequence SEQ ID NO: 1.

The present invention relates to interleukin-6 trans-signaling inhibitor for use in the treatment or prevention of a disease caused by coronavirus infection in a subject.

As used herein, the term "treating" (or "treat" or "treatment" or "therapy") refers to processes involving a slowing, interrupting, arresting, controlling, stopping, reducing, or reversing the progression or severity of an existing symptom, disorder, condition, or disease, but does not necessarily involve a total elimination of all disease-related symptoms, conditions, or disorders. Furthermore, the "treatment" also refers to a process involving the avoiding of the appearance of symptoms which have not manifested themselves yet, but which will manifest themselves due to an untreated progression of the disorder, condition or disease. The "treatment" of a disorder or disease may also lead to a partial response (e.g., amelioration of symptoms) or complete response (e.g., disappearance of symptoms) of the subject/patient suffering from the disorder or disease. Accordingly, the "treatment" of a disorder or disease may also refer to an amelioration of the disorder or disease, which may, e.g., lead to a halt in the progression of the disorder or disease or a delay in the progression of the disorder or disease. In the present invention, the condition or disorder to be treated are diseases caused by coronavirus infection.

As used herein, the term "preventing" (or "prevent" or "prevention") refers to the capacity of the interleukin-6 trans-signaling inhibitor to minimize or hinder the progression of a disease, condition or disorder in a subject.

The term "coronavirus" refers to a group of related RNA viruses that cause diseases in mammals. In humans, they cause respiratory tract infections that can range from mild to lethal. Mild illnesses in humans include some cases of the common cold, while more lethal varieties can cause severe acute respiratory syndrome (SARS), Middle East respiratory syndrome (MERS). Examples of diseases caused by coronavirus infection include, without limitation to, respiratory infections (common cold, pneumonia), lung injury and endotheliopathy affecting different organs (lung, liver, heart, brain, intestines), depression or anxiety, changes in difficulty of thinking and concentrating (referred to as "brain fog"). In people with Long COVID have symptoms similar to those reported by people with myalgia encephalomyelitis/chronic fatigue syndrome and other poorly understood chronic illnesses that may occur after other infections.

In other preferred embodiment of the invention, the interleukin-6 trans-signaling inhibitor for use in the treatment or prevention of a disease caused by coronavirus infection, wherein the disease is lung injury and/or endotheliopathy.

Particularly, in the present invention, the interleukin-6 trans-signaling inhibitor is used for the treatment or prevention of severe acute respiratory syndrome coronavirus 2 (SARS-CoV-2).

Thus, in other preferred embodiment of the invention, the interleukin-6 trans-signaling inhibitor for use in the treatment or prevention of a disease caused by coronavirus infection, wherein the coronavirus is severe acute respiratory syndrome coronavirus 2 (SARS-CoV-2).

As used herein, the term "subject" refers to mammals, include human and non-human mammals. In a particular embodiment, the subject is a human.

Thus, in other preferred embodiment of the use of the invention, the subject is a mammal, preferably is a human.

As understood by the person skilled in the art the interleukin-6 trans-signaling inhibitor can be comprised in a composition (hereinafter "the composition of the invention"), preferably a pharmaceutical composition (hereinafter "the pharmaceutical composition of the invention"), for its use in the treatment or prevention of a disease caused by coronavirus infection.

Thus, in other preferred embodiment of the use of the invention, the interleukin-6 trans-signalling inhibitor is comprised within a pharmaceutical composition.

The composition of the invention may be formulated for pharmaceutical administration, i.e., forming part of pharmaceutical products to be administered to the subject by any means of administration. Additionally, the composition of the invention may comprise one or more components or compounds having any biological and/or pharmacological useful activity in the prevention and/or treatment of a disease caused by coronavirus infection.

In other preferred embodiment, the pharmaceutical composition of the invention comprises a pharmaceutically acceptable carrier and/or an excipient.

The term "excipient" refers to a substance that helps the absorption of any components or compounds of the composition of the invention or stabilizes the components or compounds and/or assists the preparation of the pharmaceutical composition in the sense of giving it consistency or flavors to make it more pleasant. Thus, the excipients may have the function, by way of example but not limited thereto, of binding the components (for example, starches, sugars or cellulose), sweetening, coloring, protecting the active ingredient (for example, to insulate it from air and/or moisture), filling a pill, capsule or any other presentation or a disintegrating function to facilitate dissolution of the components, without excluding other excipients not listed in this paragraph. Therefore, the term "excipient" is defined as that material that included in the galenic forms, is added to the active ingredients or their associations to enable their preparation and stability, modify their organoleptic properties or determine the physical and chemical properties of the pharmaceutical composition and its bioavailability. The "pharmaceutically acceptable" excipient must allow the activity of components or compounds of the pharmaceutical composition, that is, be compatible with the effect exerted by the interleukin-6 trans-signaling inhibitor.

The "vehicle" or "carrier" is preferably an inert substance. Carrier functions are to facilitate the incorporation of other components or compounds, allow better dosage and administration and/or give consistency and form to the pharmaceutical composition. Therefore, the carrier is a substance used in the drug to dilute any of the components or compounds of the pharmaceutical composition of the present invention to a given volume or weight; or that even without diluting these components or compounds, it is able to allow better dosage and administration and/or give consistency and form to the drug. When the presentation is liquid, the pharmaceutically acceptable carrier is the diluent. The carrier can be natural or unnatural. Examples of pharmaceutically acceptable carriers include, without being limited thereto, water, salt solutions, alcohol, vegetable oils, polyethylene glycols, gelatine, lactose, starch, amylose, magnesium stearate, talc, surfactants, silicic acid, viscous paraffin, perfume oil, monoglycerides and diglycerides of fatty acids, fatty acid esters petroetrals, hydroxymethylcellulose, polyvinylpyrrolidone and the like.

Furthermore, the excipient and the carrier must be pharmacologically acceptable, i.e., the excipient and the carrier are permitted and evaluated so as not to cause damage to the subject to whom it is administered.

In each case the presentation of the composition will be adapted to the type of administration used. Thus, the composition may be presented in the form of solutions or any other form of clinically permissible administration and in a therapeutically effective amount.

The composition of the present invention may be administered by oral, parenteral (eg, intracerebral, intramuscular, intraperitoneal, intravenous, intracisternal injection or infusion, subcutaneous injection or implant, or intra-nasal administration) routes of administration, preferably formulated for intraperitoneal administration.

Thus, in other preferred embodiment of the invention, the interleukin-6 trans-signalling inhibitor or the composition is formulated for oral, intracerebral, intramuscular, intraperitoneal, intravenous, intracisternal injection or infusion, subcutaneous injection or implant, or intra-nasal administration, preferably for intraperitoneal administration.

### DESCRIPTION OF THE DRAWINGS

**Figure 1****: Experimental scheme and impact of IL-6 trans-signalling inhibition by sgp130Fc in SARS-CoV-2 infection-related mortality in k18-hACE2 mice. a)** Experimental design for the animal study. Eight-month-old male k18-hACE2 mice were divided into three groups: i) mock (non-infected) (n = 4); ii) SARS-CoV-2 infected mice injected i.p. with vehicle (PBS) (n = 17); iii) SARS-CoV-2 infected and treated i.p. with sgp130Fc at 1 mg/kg per dose (n = 18). Mice were daily monitored until 15 dpi (experimental end-point). **b)** Viral load obtained from nasal swabs at 4 dpi quantified by qPCR. Sample size (n) of each group is indicated below the corresponding bar. i) mock: non-infected; ii) SARS-CoV-2 (15 dpi): surviving infected mice injected i.p. with vehicle at the end of experiment; iii) SARS-CoV-2 + sgp130Fc (15 dpi): surviving infected mice injected i.p. with sgp130Fc at the end point of the experiment; iv) SARS-CoV-2 (< 15 dpi): dead/euthanized infected mice injected i.p. with vehicle before the scheduled endpoint of the experiment; v) SARS-CoV-2 + sgp130Fc (< 15 dpi): dead/euthanized infected mice injected i.p. with sgp130Fc before the end of the experiment. **c)** Percentage of mice survival. **d)** Univariate logistic regression analysis (Wald test). OR: odds ratio. Data are combined from two independent experiments. < 15 dpi: dead/euthanized mice before the end of the experiment. 15 dpi: surviving mice at the end of experiment. *p* values were determined by nonparametric Mann-Whitney test or by ANOVA as appropriate. **p* ≤ 0.05, ***p* ≤ 0.005.

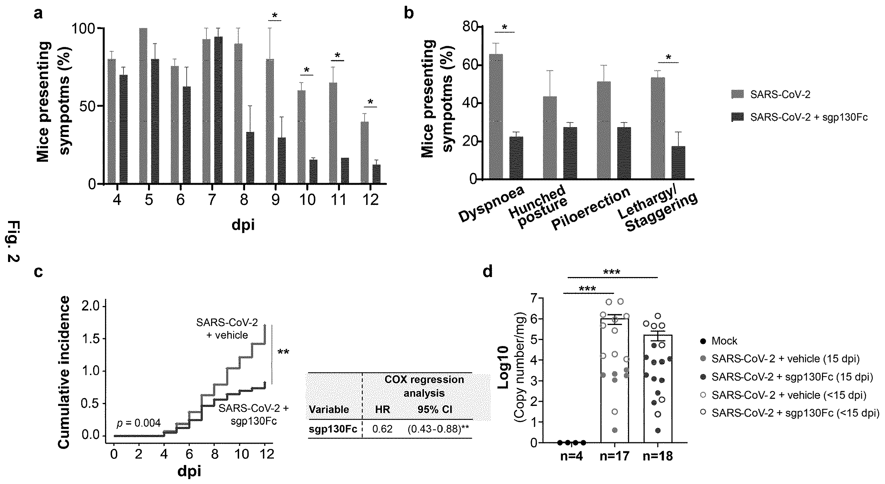

**Figure 2****: Effect of targeting IL-6 trans-signaling by sgp130Fc in symptomatology in SARS-CoV-2 infected mice.** Analysed symptoms consisted on dyspnoea, hunched posture, piloerection and lethargy/staggering. a) Percentage of mice presenting at least one of the symptoms previously described. Daily significant differences between experimental groups were determined by Chi-squared test. b) Percentage of mice presenting individual symptoms considered as positive when a mouse showed it at any day during the period of follow-up. Significant differences between experimental groups were determined by Chi-squared test. c) Cumulative incidence of symptomatology during the follow-up timeframe and COX regression hazard model for sgp130Fc in the context of symptomatology development. HR: Hazard ratio. d) Viral load in lung tissue post-mortem (< 15 dpi and 15 dpi) quantified by RT-qPCR. Sample size (n) of each group is indicated below the corresponding bar. Data are combined from two independent experiments. dpi: day post infection. < 15 dpi: dead/euthanized mice before the end of the experiment. 15 dpi: surviving mice at the end of experiment. p values were determined by nonparametric Mann-Whitney test or by ANOVA as appropriate. *p ≤0·05, **p ≤ 0 01, ***p ≤ 0 001.

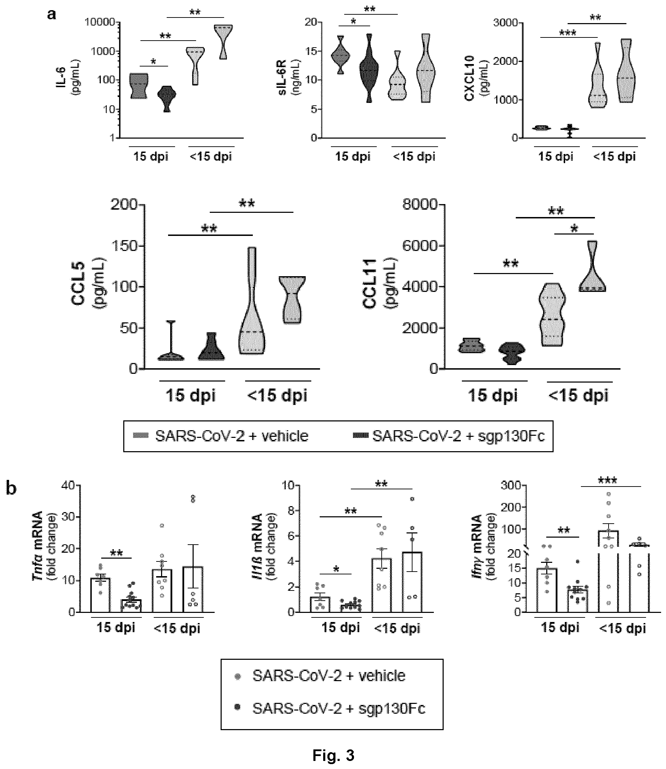

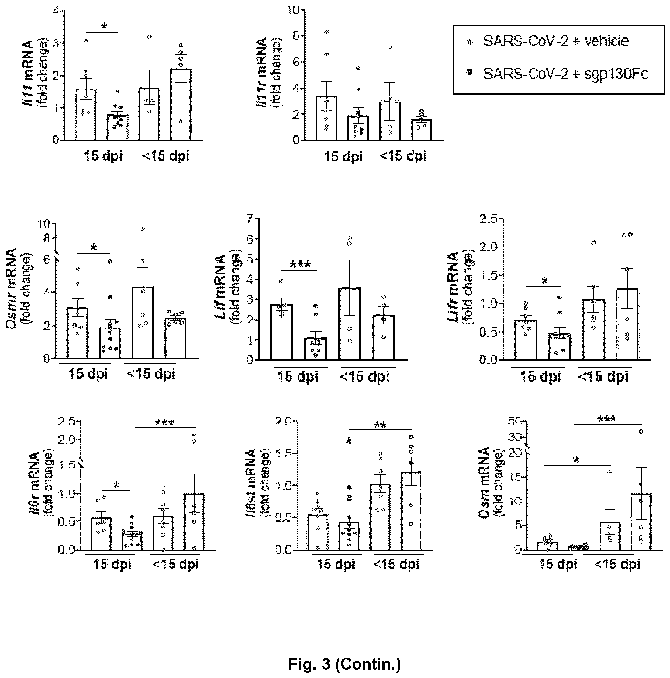

**Figure 3****: Impact of IL-6 inhibition by sgp130Fc in cytokines/chemokines levels in serum and lung tissues from SARS-CoV-2 infected mice. a)** Serum levels of the indicated proteins quantified by multiplex platform in each experimental group, surviving (15 dpi) or dead (< 15 dpi) mice. **b)** mRNA expression of the indicated cytokines and chemokines in lung tissue measured by qPCR in surviving or dead mice. Control is set to 1 for each experiment and data is presented as fold-change *vs* control (mean ± SEM). Data are combined from two independent experiments. < 15 dpi: dead/euthanized mice before the end of the experiment. 15 dpi: surviving mice at the end of experiment. *p* values were determined by nonparametric Mann-Whitney test or by ANOVA as appropriate. **p* ≤ 0.05, ***p* ≤ 0.005, ****p* ≤ 0.001.
**Figure 4****: Histopathological analysis of lungs from sgp130Fc-treated SARS-CoV-2 infected mice.** Representative images of lung sections from mice of each experimental group. a) H&E stain from dead/euthanized SARS-CoV-2-infected animals (< 15 dpi), untreated (vehicle) and sgp130Fc treated. Pictures show multifocal lesions, alveolar haemorrhage (asterisk), oedema (black arrowhead), inflammatory infiltrates (black arrow) (50 X, 200 X and 400 X). b) H&E stain from surviving SARS-CoV-2-infected animals (15 dpi), untreated and sgp130Fc-treated. Inflammatory foci (dash lines) (50 X) and cells, mainly lymphocytes (black arrow) (200 X and 400 X) are indicated. c) Masson's trichrome stain from surviving SARS-CoV-2-infected animals (15 dpi), untreated and sgp130Fc-treated (50 X). Deposits of connective tissue interposed between lymphocytes (black arrow) (200 X and 400 X). d) Number of inflammatory foci per mouse and % of inflammatory foci area per lung section area quantified by ImageJ/FIJI software in surviving mice (15 dpi), p values were determined by nonparametric Mann-Whitney test. *p ≤ 0 05. e) H&E stain from uninfected animals (mock; 50 X, 200 X and 400 X). H&E: Haematoxylin Eosin staining. Data are combined from two independent experiments. dpi: day post infection. < 15 dpi: dead/euthanized mice before the end of the experiment. 15 dpi: surviving mice at the end of experiment.

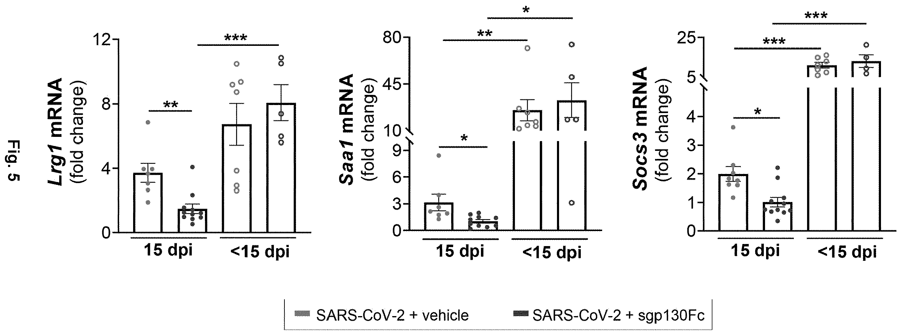

**Figure 5****: STAT3 target genes expression in lung tissue from sgp130Fc-treated SARS-CoV-2 infected mice.** mRNA expression of *Lrg1, Saa1* and *Socs3* in lungs quantified by qPCR. Control is set to 1 for each experiment and data presented as fold-change vs control. Data are combined from two independent experiments. < 15 dpi: dead/euthanized mice before the end of the experiment. 15 dpi: surviving mice at the end of experiment. *p* values were determined by nonparametric Mann-Whitney test or by ANOVA as appropriate. **p* ≤ 0.05, ***p* ≤ 0.005, ****p* ≤ 0.001.

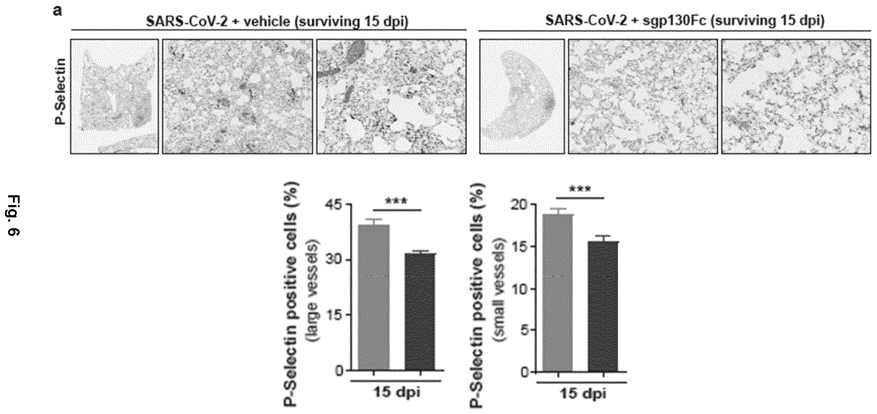

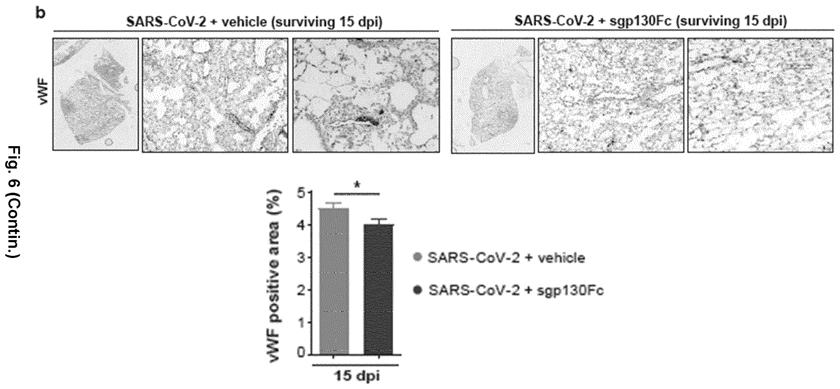

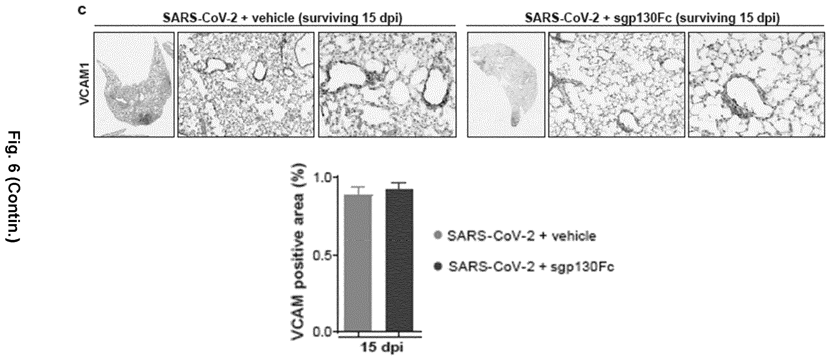

**Figure 6****: Impact of IL-6 trans-signaling inhibition by sgp130Fc on endothelial cells activation in lungs from SARS-CoV-2 infected surviving mice (15 dpi).** Representative immunostaining images of lung sections from surviving mice. **a)** Immunoreaction of P-selectin on luminal surface of endothelial cells and platelet aggregates (50 X, 200 X and 400 X). Positive cells were quantified using QuPath software. Data are presented as % of positive cells (mean ± SEM). **b)** vWF immunostaining in mural thrombus, oedematous fluid in alveoli and endothelial cells. Positive area was quantified using ImageJ/FIJI. Data are presented as mean ± SEM **c)** Immunoreaction of VCAM1 on vascular endothelial cells (50 X, 200 X and 400 X). Data are presented as mean ± SEM. 15 dpi: surviving mice at the end of experiment.

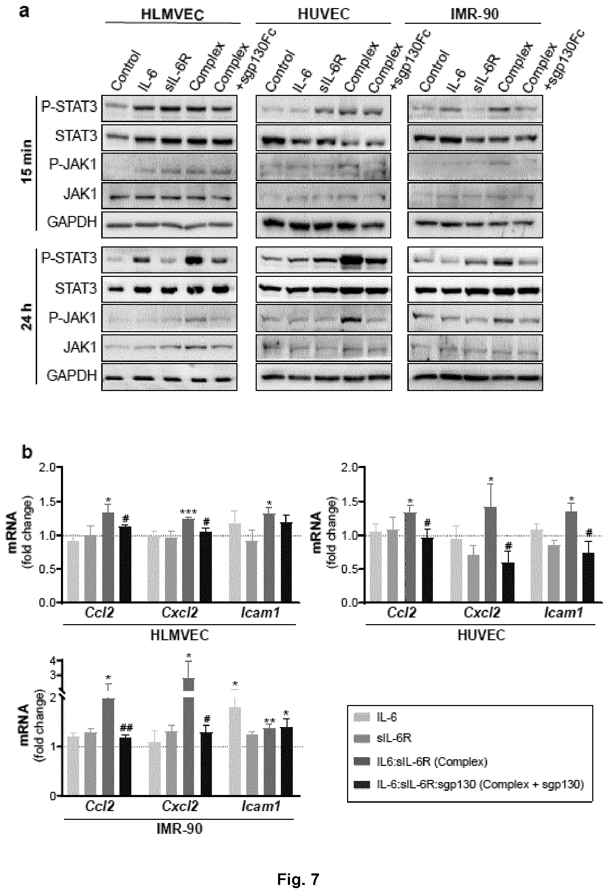

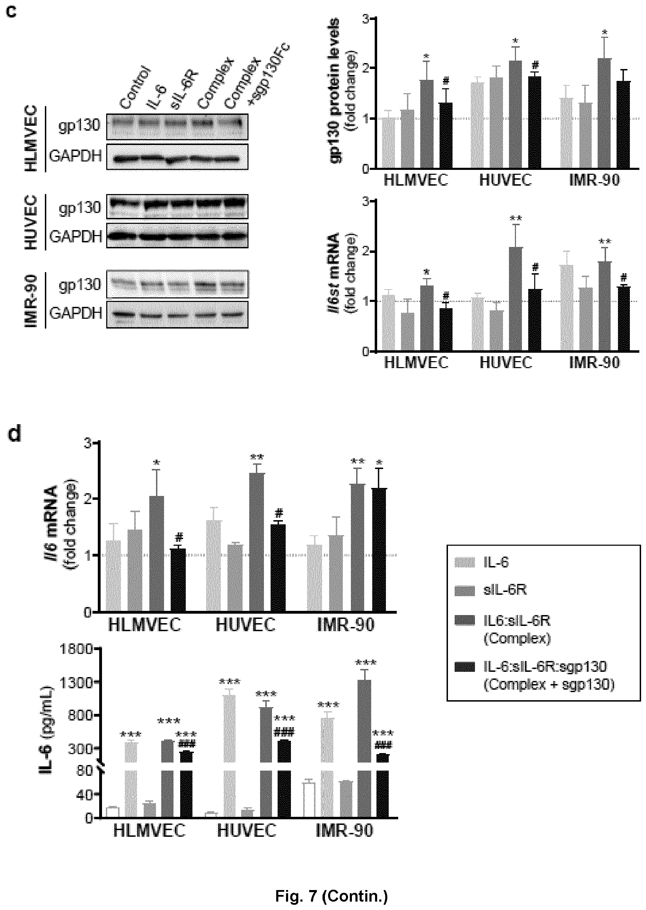

**Figure 7****: Effect of IL-6 trans-signaling on human endothelial cells and lung fibroblasts.** Human lung microvascular cells (HLMVEC), human umbilical vein endothelial cells (HUVEC) and human lung fibroblasts (IMR-90) were treated with vehicle (control), IL-6 (20 ng/mL), IL-6R (20 ng/mL) or IL-6:sIL-6R (Complex, 20 ng/mL) in the presence or absence of sgp130Fc (300 ng/mL). a) Representative Western blot of P(Tyr705)-STAT3, STAT3, P(Tr1034/1035)-JAK1 and JAK1 from cell lysates after 15 min and 24 h of treatment. GAPDH was used as loading control. b) mRNA expression of proinflammatory marker (Ccl2, Cxcl2 and Icam1) by qPCR after 24 h of treatment. c) Representative Western blot, densitometric analysis and mRNA expression by qPCR of gp130 after 24 h of treatment. GAPDH was used as loading control. d) ll6 mRNA expression by qPCR and IL-6 levels by ELISA in supernatants from cultured cells after 24 h of treatment. Data are combined from 3 independent experiments. Control is set to 1 for each experiment and data presented as fold-change vs control (mean ± SEM). Complex: IL-6:sIL-6R. Complex + sgp130Fc: IL-6:sIL-6R:sgp130Fc. p values were determined by nonparametric Mann-Whitney test or by ANOVA as appropriate. *p ≤ 0·05, **p ≤ 0 01, ***p ≤ 0 001 compared to control. #p ≤ 0·05, ##p ≤ 0 01, ###p ≤ 0 001 compared to complex treatment.

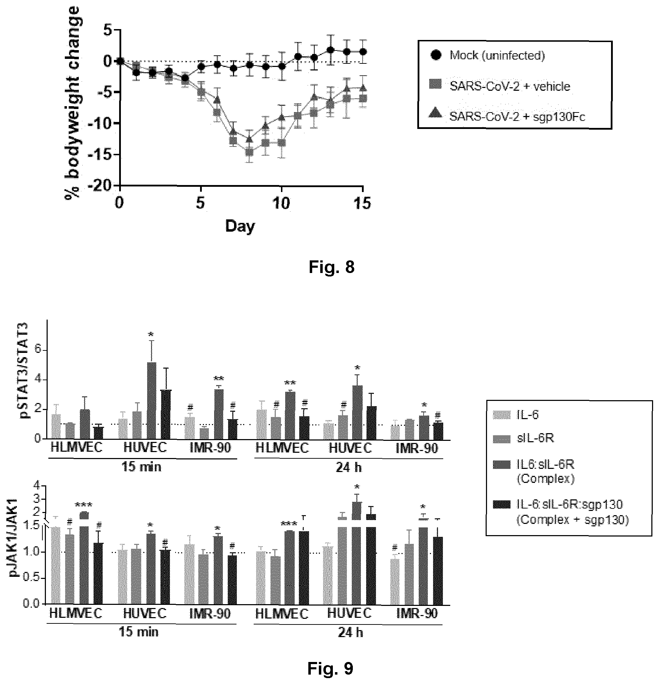

**Figure 8****. Percentage of body weight change in SARS-CoV-2 K18-hACE2 infected mice.** SARS-CoV-2 infected mice displayed body weight change starting at 5 dpi, without differences between the two groups: infected and untreated (vehicle) group vs infected and sgp130Fc-treated group. Data are shown as mean ± SEM. Data are combined from two independent experiments.
**Figure 9****. Western blots densitometric analyses of P(Tyr705)-STAT3/STAT3 and P(Tyr1034/1035)-JAK1/JAK1.** Phosphorylated/total protein ratio was determined by densitometric analysis. Human lung microvascular cells (HLMVEC), human umbilical vein endothelial cells (HUVEC) and primary lung fibroblast (IMR-90) were treated with vehicle, IL-6 (20 ng/mL), IL-6R (20 ng/mL) or IL-6:sIL-6R complex (20 ng/mL) in the presence or absence of sgp130Fc (300 ng/mL) for 15 min and 24 h. GAPDH was used as loading control. Data are combined from 3 individual experiments. Control is set to 1 for each experiment and data presented as fold-change vs control (mean ± SEM). Images are representative of three independent experiments. Complex: IL-6:sIL-6R complex. Complex + sgp130: IL-6:sIL-6R:sgp130 complex

### Examples

### Materials and Methods

### Mice and ethics statement

K18-hACE2 humanized mice (SN34860-B6.Cg-Tg(K18-ACE2)2Prlmn/J) were obtained from The Jackson Laboratory. This mouse strain has been previously used as a model for SARS-CoV-2 -induced disease and it presents signs of diseases, and biochemical and lung pathological changes compatible with the human disease (Dong et al., J Verol. 2022 Jan 12;96(1):e0096421). Mice had access to water and food ad *libitum.* All animal care and procedures were carried out conformed to the Guide for the Care and Use of Laboratory Animals published by the National Academy of Sciences in accordance with the Spanish legislation (BOE 34/2013), EU Directive 2010/63/EU for animal experiments, and were approved by the animal ethics committee of Comunidad de Madrid (PROEX 087.0/21).

### Virus and sgp 130Fc

SARS-CoV-2 virus (isolate Navarra-2473) was obtained from nasopharyngeal swab of a COVID-19 patient hospitalized in the University of Navarra Clinic (Pamplona, Spain) in April 2020, after obtaining written informed consent and acquiring the necessary Regional Government permits. Initially, the virus sample was used to infect Vero-E6 cells (ATCC^{®} CRL-1586^{™}) which were grown with Eagle's Minimum Essential Medium (MEM) containing 10 % fetal bovine serum (FBS) and antibiotics.

Cell supernatants were collected at 48 h post-inoculation and titrated using a lysis plate assay in Vero-E6 cell monolayers, resulting in a titter of 4.3 × 10⁷ plaque forming units (PFU) /ml. The viral stock was quickly frozen in a methanol-dry ice bath and stored at -80°C. For subsequent experiments, smaller aliquots of the original stock were prepared, obtaining a titter of 1.8 × 10⁷ PFU/ml after being frozen again.

Soluble gp130Fc (sgp-130Fc) is a fusion protein generated by linking the entire extracellular portion of human gp130 to the Fc portion of human IgG1 (SEQ ID NO: 1). The protein bound IL-6 only in the presence of sIL-6R and consequently specifically inhibits IL-6 trans-signaling without affecting classic signaling.

### Animal experiments

8-month-old male k18-hACE2 transgenic mice were inoculated intranasally with 25µL modified Eagle medium (MEM) containing 1 X 10⁴ PFU of SARS-CoV-2. Mice were randomly distributed in three groups: Control (mock, non-infected, n = 4), SARS-CoV-2-infected (n = 17) and SARS-CoV-2-infected sgp130Fc-treated mice (n = 18). gp130Fc at the dose of 1 mg/kg was administered by intraperitoneal injection (i.p.) twice: at 2 and 8 days after virus inoculation. Mock and untreated infected animals received PBS (vehicle) i.p.

Mouse weight and health were monitored daily, up to 15 days post infection (dpi) (end of experiment) or until they reached end-point criteria (body weight loss > 20% or > 13%-16% plus other symptoms such as dyspnoea, hunched posture, lethargy/staggering, and piloerection). All experiments with SARS-CoV-2 were performed in a Biosafety level 3 (BSL3) Laboratory at the Veterinary Health Surveillance Center (VISAVET, Complutense University of Madrid).

### Lung extraction

The right lung was inflated with formalin (4%) and fixed with the same buffer for 7 days before being embedded in paraffin for histological analysis. The left lung lobe was used for RNA extraction (cellular and viral), as described below.

### RNA isolation, reverse transcription and gene expression analysis

For RNA extraction, lung tissue samples were incubated with RNAlater, and subsequently frozen at -80°C or processed directly according to the Rneasy Plus Micro kit (Qiagen, Hilden, Germany) protocol. For extraction of viral RNA by nasal swabs, samples were collected at 4 dpi and processed using a MagMax core kit (Applied Biosystems, Woburn, MA, USA) according to the manufacturer instructions. Quantitative real-time PCR (qPCR) in lungs and swabs to specifically detect SARS-CoV-2 RNA was performed according to the indications of the COVID-19 dtec-459 RT-qPCR test kit (F100 format, Genetic PCR Solutions, Orihuela, Spain). For detection of cytokines/chemokines mRNA expression, cDNA was obtained by PrimeScript RT from Takara Clontech (Takara Biomedical Technology, Beijing, China). qPCR was performed using LightCycler 96 system thermocycler and SYBR Green (Roche, Basel, Switzerland). Product quantities were calculated by applying the ΔCt method ΔCt = (Ct of gene of interest - Ct of housekeeping genes). The housekeeping genes used for input normalization were cyclophilin or RPLO. Primers for qPCR are listed in Table 1.

**Table 1. List of primers for qPCR.**

| Gene | Sequence (5' - 3') | Gene | Sequence (5' - 3') |
|---|---|---|---|
| mCyclophilin | Fw: | mhRplo | Fw: |
| | GTCTCCTTCGAG CTGTTTGC (SEQ ID NO: 2) | | AACATCTCCCCCTTCTCC TT (SEQ ID NO: 24) |
| | Rv: | | Rv: |
| | CGTGTAAAGTCA CCACCCTG (SEQ ID NO: 3) | | GAAGGCCTTGACCTTTTC AG (SEQ ID NO: 25) |
| mll6r | Fw: | mll6st | Fw: |
| | ACGAAGGCTGTG CTTTGC (SEQ ID NO: 4) | | AAGGATTTGGCTAGCGC AAGC (SEQ ID NO: 26) |
| | Rv: | | Rv: |
| | TTCGTTGTGGCT GGACTTGC (SEQ ID NO: 5) | | CCACACGATGTAGCTGG CATT (SEQ ID NO: 27) |
| mOsm | Fw: | mOsmr | Fw: |
| | ACGGCTTCTAAG AACACTGC (SEQ ID NO: 6) | | TCTTGGAGGAGCCTTTAC CAT (SEQ ID NO: 28) |
| | Rv: | | Rv: |
| | GATATAGGGCTC CAAGAGTG (SEQ ID NO: 7) | | TTTCCAGGAACTCCAGTT GC (SEQ ID NO: 29) |
| mLif | Fw: | mLifr | Fw: |
| | CGAGACCACGCT CCAGTATAT (SEQ ID NO: 8) | | CGATGCATGCAAACGGT CTGA (SEQ ID NO: 30) |
| | Rv: | | Rv: |
| | TTTCCAGTGCAG AACCAGCAG (SEQ ID NO: 9) | | GACTTCGTGATCACCAG GTGA (SEQ ID NO: 31) |
| mll11 | Fw: | mll11r | Fw: |
| | ACTAGCTGCACA GATGAGAG (SEQ ID NO: 10) | | CCTGGAGACATCTGTCCT CA (SEQ ID NO: 32) |
| | Rv: | | Rv: |
| | GCTCCAGAGTCT TTAGGGAA (SEQ ID NO: 11) | | CCTGAGCAGCTGCTGCT CAT (SEQ ID NO: 33) |
| mSocs3 | Fw: | mSaa1 | Fw: |
| | AAGGCCGGAGAT TTCGCT (SEQ ID NO: 12) | | TTGTTCACGAGGCTTTCC (SEQ ID NO: 34) |
| | Rv: | | Rv: |
| | AACTTGCTGTGG GTGACCAT (SEQ ID NO: 13) | | TGAGCAGCATCATAGTTC C (SEQ ID NO: 35) |
| mLrg1 | Fw: | mTnfα | Fw: |
| | TAGAGGAGCAGC TATGGTCTCT (SEQ ID NO: 14) [?] | | AGCCCACGTCGTAGCAA ACC (SEQ ID NO: 36) |
| | Rv: | | Rv: |
| | TACCCTCAGCCG ACTGCAGTAT (SEQ ID NO: 15) | | GAGGAGCACGTAGTCGG GGC (SEQ ID NO: 37) |
| mll1β | Fw: | mIfnγ | Fw: |
| | CTTGGGATCCAC ACTCTCCAG (SEQ ID NO: 16) | | ACAGCAAGGCGAAAAAG GA (SEQ ID NO: 38) |
| | Rv: | | Rv: |
| | AAATACCTGTGG CCTTGGGC (SEQ ID NO: 17) | | TGGTGGACCACTCGGAT GA (SEQ ID NO: 39) |
| hCyclophilin | Fw: | hll6 | Fw: |
| | AGACAAGGTCCC AAAGACAGC (SEQ ID NO: 18) | | TGACCCAACCACAAATGC (SEQ ID NO: 40) |
| | Rv: | | Rv: |
| | CAGTGCCATTAT GGCGTGTGA (SEQ ID NO: 19) | | CTGGCTCTGAAACAAAG GAT (SEQ ID NO: 41) |
| hgp130 | Fw: | hCcl2 | Fw: |
| | TTGACGTTGCAG ACTTGGCT (SEQ ID NO: 20) | | GCAAGTGTCCCAAAGAA GCT (SEQ ID NO: 42) |
| | Rv: | | Rv: |
| | AACTGGAGATTC AGGACTGA (SEQ ID NO: 21) | | AGCTTCTTTGGGACACTT GC (SEQ ID NO: 43) |
| hCxcl2 | Fw: | hlcam | Fw: |
| | GCAGAAAGCTTG TCTCAACCC (SEQ ID NO: 22) [?] | | AGGATGGCACTTTCCCA CTG (SEQ ID NO: 44) |
| | Rv: | | Rv: |
| | GGCAGAAAGCTT GTCTCAACCC (SEQ ID NO: 23) | | GGAGAGCACATTCACGG TCA (SEQ ID NO: 45) |

### Serum cytokine and chemokine levels

Serum levels of IL-6, sIL-6R, CXCL10, CCL5 and CCL11 were analysed using two pre-configured multiplex panels (Mouse Cytokine/Chemokine Magnetic Bead Panel Cat. # MCYTOMAG-70K, -70K-PMX, -70K-PX32, 70PMX25BK and 70PMX32BK; Mouse Soluble Cytokine Receptor Magnetic Bead Panel Cat. # MSCRMAG-42K). The experiment was performed in a Bio-Plex 200 System (Bio-Rad Laboratories, Inc., Hercules, CA, USA) at the Investigation, Technology and Innovation Center (CITIUS) of the University of Seville.

### Histological processing

Lung fixed tissue sections (3 µm) from mice of each experimental group were obtained to perform Haematoxylin-Eosin (H&E), Masson's Trichrome staining and immunohistochemistry. P-selectin (CD62P) (Ref. ab255822) and vascular cell adhesion molecule-1 (VCAM-1) (Ref. ab134047) antibodies were from Abcam (Cambridge, UK). Von Willebrand factor (vWF) antibody (Ref. BS-100448R) was from Thermo Fisher Scientific. PolyStain 2-Step Plus Kit, HRP for 3,30-diaminobenzidine tetrahydrochloride DAB, Rabbit (NB-23-00050-3, NeoBiotec, Nanterre, France) was used to enhance antibody signal. Nuclei was stained using Mayer's haematoxylin. Images were captured using Olympus Bx-61 (Olympus, Tokyo, Japan) and Thunder Dmi8 (Leica, Wetzlar, Germany) microscopes.

### IHC imaging analysis: P-Selectin, vWF and VCAM-1 quantification

Expression of P-selectin was quantified. 5-7 histological sections were analysed for each mice of the different experimental groups, and five hot-spot regions per section were chosen using an area of 416000µm² with QuPath software. Areas with artefacts, such as tissue-tear, folding or background noise were excluded. Quantification of P-selectin positive cells were performed independently in large and small vessels. The values were obtained as percentage of positive cells. The vWF and VCAM-1 positive area in each specimen were quantified in 4 randomly selected regions in an unbiased fashion using Fiji (ImageJ).

### Primary human cell cultures

Human lung microvascular endothelial cells (HMVEC-L-Lung MV Endo, EGM-2MV, CC-2527, Lonza, Basel, Switzerland) were cultured using CC-3202 EGM-2 MV BulletKit (CC-3156_CC-4147, Lonza). Human umbilical vein endothelial cells (HUVEC) (CC-2517, Lonza) were cultured using H3CC-3124 medium (EGM TM BulletKit TM (CC-3124), Lonza). Human primary lung IMR90 fibroblasts (ATCC^{®} CCL186^{™}, ATCC) were cultured using Eagle's (MEM) supplemented with FBS 10 %. Cells were cultured in 5% CO₂ at 37°C. Recombinant human proteins were purchased from R&D Systems (Minneapolis, MN, USA): IL-6 (Ref. 206-IL-05/CF); IL-6 Rα (Ref. 227-SR-025); gp130 Fc Chimera (Ref. 671-GP-100). Cells, at passage 3-6, were cultured in serum-free medium overnight and then stimulated by IL-6 (20 ng/mL), sIL-6R (20 ng/mL), and IL-6:sIL-6R (20 ng/mL) complex in the presence or absence of sgp130Fc (300 ng/mL) for 15 min, 24 h or 48 h.

### Western blots

Cell lysates were obtained using lysis buffer (150 mM NaCl, 1 mM EDTA, 25 mM Tris (pH 7.5), 1 mM orthovanadate, 1% triton, and protease inhibitor cocktail (Roche)). Protein concentration was determined by DC protein assay (Bio-Rad). Proteins were separated 7.5%-12% SDS-PAGE in the presence of a reducing agent (2-mercaptoethanol) and were transferred to polyvinylidene difluoride (PVDF) membranes (Bio-Rad) and stained with Ponceau red solution (Sigma-Aldrich, San Louis, MO, USA). Primary antibodies against P-STAT-3(Tyr705) (Ref. 9131S), STAT-3 (Ref. 30835S), P-JAK1(Tr1034/1035) (Ref. 74129S), JAK-1 (Ref. 3344T) were purchased from Cell Signalling (Danvers, MA, USA); gp130 (Ref. sc-656) from Santa Cruz Bt. (Santa Cruz, CA, USA); GADPH (Ref. 1461) from StemCell Tech (Vancouver, Canada). Horseradish peroxidase-conjugated secondary antibodies (GE Healthcare, Chicago, IL, USA) were used. The proteins were detected with enhanced chemiluminescence (Ref. 1705061, Bio-Rad) and the densitometric analysis was performed using the software Image Lab 6.0 (BioRad).

### Statistical analysis

Analyses were performed with GraphPad Prism 8.4.2, IBM SPSS Statistics 22 software and R studio i386 4.1.2. Data are expressed as mean ± SEM. Sample normality was screened using Kolmogorov-Smirnoff and Shapiro-Wilk test. Comparisons between the values for different variables were analysed by one-way ANOVA followed by Student's t test, Man-Whitney U-test and Chi-square test of independence, were performed as indicated at each figure legend. Overall, a p value < 0.05 was considered statistically significant. Cumulative incidence was performed by Kaplan-Meier analysis, and statistical significance was determined by log-rank test. COX regression hazards model was used to estimate the hazard of symptoms development.

### Example 1: IL-6 trans-signaling inhibition by sgp130Fc reduces clinical outcomes in an experimental model of COVID-19

To investigate IL-6 trans-signaling inhibition by sgp130Fc in COVID-19, the inventors used k18-hACE2 transgenic mice intranasally infected with a sub-lethal dose of SARS-CoV-2 (10⁴ PFU); dose has been previously validated. Infected animals were divided into 2 groups: i) untreated mice injected i.p. with PBS (vehicle) and ii) treated mice, received sgp130Fc (1 mg/kg) i.p. twice, at 2- and 8-days post-infection (dpi). K18-hACE2 uninfected mice served as controls (mock). Mice were monitored daily until 15 dpi, the scheduled endpoint of the experiment (Fig. 1a). Infected mice that presented end-point criteria (body weight loss > 20% or >13%-16% plus other symptoms such as dyspnoea, hunched posture, lethargy/staggering, and piloerection) were euthanized. The viral load was detected by nasal swab at 4 dpi in all mice, without differences between non-treated and sgp-130Fc-treated groups (Fig. 1b). Importantly, the survival rate was significantly superior in sgp130Fc-treated mice compared to untreated animals (Fig. 1c). In infected group, the proportion of untreated mice that died or were euthanized before the scheduled endpoint of the experiment (< 15 dpi) was significantly higher (55.18 %) than those that received sgp130Fc treatment (27.8 %). Univariate WALD test revealed that treated mice were 33.9 % less likely to die than non-treated animals (Fig. 1d).

In line with previous results, SARS-CoV-2 infected mice displayed body weight change starting at 5 dpi, without differences between the two groups (Fig. 8). However, when the inventors assessed other clinical symptoms such as dyspnoea, hunched posture, lethargy/staggering and piloerection, the inventors observed a significant reduction in the number of animals presenting at least one of these symptoms in the treated group compared to untreated from 8 dpi onwards (Fig. 2a). Analysis of individual symptoms considered as positive when a mouse showed it at any day during the period of follow-up, revealed that sgp130Fc treatment reduced the percentage of animals presenting dyspnoea, hunched postured, piloerection and lethargy/staggering (Fig. 2b). The cumulative symptomatology incidence was significant lower in the treated group and COX regression analyses revealed that sgp130 treatment had a protective role against development of symptoms (HR 0.62) (Fig. 2c). Since lungs are one of the main SARS-CoV-2 target organs, presence of viral RNA was analysed post-mortem in all animals. As expected, viral load was significantly increased in infected animals, while non-infected mice resulted in absence of viral RNA. Importantly, no differences were found between treated vs non-treated animals neither survivors nor dead/euthanized mice (Fig. 2d). Overall the findings indicate that targetting IL-6 trans-signaling by sgp130Fc reduces SARS-CoV-2 infection associated mortality as well as severity of the symptoms in surviving mice (15 dpi).

### Example 2: sgp130Fc decreases the expression of proinflammatory cytokines/chemokines in SARS-CoV-2- infected mice

Lung inflammation is one of the main causes of life-threatening respiratory disorders in severe forms of COVID-19. To identify the inflammatory mediators involved in the pathogenesis of SARS-CoV-2 as well as the potential impact of the specific blockade of IL-6 trans-signaling, the inventors measured the protein levels of cytokines and chemokines both in serum and lung homogenates. Infected dead mice (< 15 dpi) showed higher systemic levels of IL-6, CXCL10 (IP10), CCL5 (Rantes) and CCL11 (Eotaxin) than surviving mice (15 dpi). In contrast, in the untreated group levels of slL-6R were significantly reduced in dead compared to surviving mice (Fig. 3a), as it has been reported in fatal outcome (exitus) of COVID-19. Nevertheless, surviving sgp130Fc-treated mice had lower levels of both, IL-6 and sIL-6R, than alive untreated mice (Fig. 3a). These observations are in concordance with previous results obtained from moderate COVID-19 patients, where increased levels of both proteins were indicators of severity.

To determine the impact of the treatment in SARS-Cov-2 infected mice, the inventors determined mRNA expression of cytokines and chemokines associated to disease progression in lung homogenates. As expected, higher levels of pro-inflammatory genes were detected in non-survivors (< 15 dpi). Notably, when the inventors focused on infected surviving mice (15 dpi), those that received sgp130Fc showed significant decreased mRNA expression of pro-inflammatory cytokines (Tnfα, Il1β and Ifny), as well as IL-6 family of cytokines and receptors related with severe COVID-19: II6r, Il6st, Osm, Osmr, Lif, Lifr, II11 and II11r (Fig. 3b). Collectively, these data suggest that sgp130Fc treatment reduces the expression of inflammatory markers associated to disease progression, in line with the milder virus-derived symptoms presented by these mice.

### Example 3: Targeting IL-6 trans-signaling by sgp130Fc ameliorates lung damage in surviving SARS-CoV-2-infected mice

The inventors next examined the effect of IL-6-trans-signalling inhibition in lungs from SARS-CoV-2 infected mice by histological analyses. Macroscopic inspection of the lungs from dead/euthanized mice (< 15 dpi) showed that all infected mice displayed alterations, including haemorrhagic, oedematous, or congestive areas, without differences between treated and untreated animals. Histologic examination of the lungs from mice of each experimental group was carried out to determine whether specific inhibition of IL-6 trans-signaling affected disease severity. SARS-CoV-2 infected dead/euthanized mice (< 15 dpi), both treated and untreated, showed similar histopathological characteristics. The inventors noticed an extensive injury in the lung tissue characterized by multifocal lesions with a tendency to confluence, alveolar oedema and intense haemorrhage. A significant interstitial leukocyte infiltration with the presence of neutrophils and mononuclear inflammation was observed in the expanding alveolar septal walls, prominently surrounding vascular structures and filling alveolar spaces (Fig. 4a). By contrast, the histopathological findings in the infected surviving mice (15 dpi) showed differences between untreated and sgp130Fc-treated mice. The former group presented a moderate to severe lymphocytic, multifocal, chronic bronchiole-interstitial pneumonia with inflammatory infiltrate cells, mainly lymphocytes and some histiocytes with the presence of alveolitis with neutrophils, macrophages and syncytial cells in a multifocal pattern. Surviving sgp130Fc-treated mice (15 dpi) displayed a more moderate interstitial pattern with a reduction of lung cell infiltration, as well as less deposits of connective tissue interspersed with lymphocytes, in comparison to untreated mice (Fig. 4b,c). Areas of dense inflammation, both individual foci as well as areas of intense inflammation produced by coalescence of numerous individual foci, were quantified. In surviving mice (15 dpi), sgp130Fc treatment resulted in a significant decrease of both number of inflammatory foci per animal (37% of reduction) as well as areas of lung inflammation (30% of reduction) compared to untreated group (Fig. 4d). As expected, lungs from non-infected mice did not show histological alterations (Fig. 4e).

To determine the effectiveness of sgp130Fc treatment, the inventors assessed STAT3 target genes mRNA: *Lrg1* (leucine rich alpha-2-glycoprotein 1), *Saa1* (serum amyloid a 1) and *Socs3* (suppressor of cytokine signaling 3) in lung samples from SARS-CoV-2 infected mice. A significant overexpression of those genes in dead/euthanized mice (< 15 dpi) was observed, both in treated and untreated groups. Focusing on survivors (15 dpi), STAT3 dependent genes were significantly downregulated in lungs from sgp130Fc-treated vs non-treated mice (Fig. 5). The results indicate that STAT3 is crucially involved in the mechanism of action of sgp130Fc and responsible for the clinical outcomes.

Inflammatory processes play an important role in the activation of endothelial cells (directly or by immune-mediated manner), being key players in lung inflammation, oedema, and disseminated coagulation with an important role in the development of COVID-19. Immunohistochemically analysis of adhesion molecules revealed a highly significant upregulation of CD62P (P-selectin), a biomarker of endothelial cell degranulation and platelet activation, in all SARS-CoV-2 infected mice. P-selectin staining revealed strong membranous/cytoplasmic staining of platelet aggregates intimately adhered to the luminal surface of lung blood vessels, representing activated platelets. It has been reported upregulation of this marker in the vascular phase of COVID-19, indicating endothelial and platelet activation as the first pathophysiologic event in COVID-19. Surprisingly, the inventors observed a marked staining of CD62P in surviving mice (15 dpi) on the luminal surface of endothelial cells and platelet aggregates with significant increased levels in untreated in comparison to sgp130Fc-treated mice in large and microcirculatory vessels (Fig. 6a). Other endothelial cell activation markers described in COVID-19, including VCAM1 and vWF were analysed. vWF was found in the endothelium of large and microcirculatory vessels as well as in the oedematous fluid and thrombi (Fig. 6b). VCAM1 expression was found in endothelial cell mainly from large vessels (Fig. 6c). Collectively, these findings indicate that lung damage was more intense in infected mice that died or had to be euthanized in the first 7-8 days after infection, without differences between treated and untreated mice. Importantly, in survivors, sgp130Fc treatment reduced lung injury, with a significant decrease of inflammatory infiltrate and markers of endothelial cell activation. In summary, targeting IL-6 trans-signaling mitigated lung injury in the SARS-CoV-2-infected mice and it could represent a novel therapy with impact in long term consequences of COVID-19.

### Example 4: sgp130Fc inhibits the effects of IL-6 trans-signaling on human primary endothelial cells and human fibroblasts

In humans, it has been described that the release of inflammatory cytokines after severe SARS-CoV-2 infection leads to cytokine storm, with extensive endothelial cell activation, tight junction barrier disruption, pulmonary hypertension, and lung fibrosis. The inventors assessed the role of IL-6 trans-signaling in human lung microvascular endothelial cells (HLMVEC), human umbilical vein endothelial cells (HUVEC) and human lung fibroblast (IMR90).

Cells were treated with IL-6 alone or with the IL-6:sIL-6R complex (trans-signaling) as described in methods for 15 min and 24 h. The inventors found that JAK1/STAT3 activation by IL-6:sIL-6R complex was strong and persisting (24 h) (Fig. 7a and Fig.9). To further confirm the causative role of IL-6 trans-signaling, the inventors used sgp130Fc. As shown in figure 7a, sgp130Fc partially blocked phosphorylation of JAK1 and the downstream STAT3. To note, increased mRNA expression of markers of inflammation/adhesion such as Ccl2 (Mcp1), Cxcl2 and Icam1 were induced by the IL-6:sIL-6R complex at 24 h and partially blocked by sgp130Fc in all cells (Fig. 7b). Since endothelial cells and fibroblasts have scant amount of functional IL-6, the extent of trans-signaling could depend on the amount of gp130. The inventors observed that gp130 was significantly upregulated at 24 h, both at mRNA and protein level, by IL-6 trans-signaling (Fig. 7c). Furthermore, this overexpression was accompanied by increment of II6 mRNA expression and IL-6 secretion (Fig. 7d). Altogether, IL-6 trans-signaling could activate an autocrine loop mainly due to the increase of gp130 and IL-6 levels, as it has been reported in VSMC and recently in endothelial cells.

## Claims

1. An interleukin-6 trans-signalling inhibitor for use in the treatment or prevention of a disease caused by coronavirus in a subject, wherein the Interleukin-6 trans-signaling inhibitor is a fusion protein comprising, in the sense N'terminal to C'terminal, the extracellular region of the glycoprotein 130 and a Fc portion of an IgG antibody.

2. The interleukin-6 trans-signalling inhibitor for use according to claim 1, wherein said interleukin-6 trans-signalling inhibitor is a fusion protein comprising an amino acid sequence having a sequence identity of at least 80% with the sequence SEQ ID NO: 1.

3. The interleukin-6 trans-signalling inhibitor for use according to claim 1 or 2, wherein the interleukin-6 trans-signalling inhibitor consists of the amino acid sequence SEQ ID NO: 1.

4. The interleukin-6 trans-signalling inhibitor for use according to any one of claims claim 1 to 3, wherein the coronavirus is SARS-CoV-2.

5. The interleukin-6 trans-signalling inhibitor for use according to any one of claims 1 to 4, wherein the disease is lung injury and/or endotheliopathy.

6. The interleukin-6 trans-signalling inhibitor for use according to any one of claims 1 to 5, wherein the subject is a mammal, preferably is a human.

7. The interleukin-6 trans-signalling inhibitor for use according to any one of claims 1 to 6, wherein the interleukin-6 trans-signalling inhibitor is comprised within a pharmaceutical composition.

8. The interleukin-6 trans-signalling inhibitor for use according to claim 7, wherein the pharmaceutical composition comprises a pharmaceutically acceptable carrier and/or an excipient.

9. The interleukin-6 trans-signalling inhibitor for use according to any one of claims 1 to 8, wherein the interleukin-6 trans-signalling inhibitor or the pharmaceutical composition is formulated for subcutaneous, intracerebral, intraperitoneal, or intranasal administration, preferably for intraperitoneal administration.
